# EUROPÄISCHE PATENTSCHRIFT

(11) **EP 0 387 250 B1**
(45) Veröffentlichungstag und Bekanntmachung des Hinweises auf die Patenterteilung: **24.11.1993**
(21) Anmeldenummer: 88907271.6
(22) Anmeldetag: 03.08.1988
(51) Int. Cl.: A61L 2/26

(54) **VENTIL FÜR EINEN STERILISIERBEHÄLTER**
VALVE FOR STERILIZATION CONTAINER
SOUPAPE POUR RECIPIENTS DE STERILISATION

(30) Priorität: 28.08.1987 DE 8711702 U; 28.08.1987 DE 8711703 U; 29.06.1988 DE 8808353 U
(43) Veröffentlichungstag der Anmeldung: 19.09.1990
(73) Patentinhaber: Wagner GmbH Fabrik für medizinische Geräte, 80634 München (DE)
(72) Erfinder: WAGNER, Peter, D-8130 Starnberg (DE)
(74) Vertreter: Koch, Günther, Dipl.-Ing.
(86) Internationale Anmeldenummer: EP8800703
(87) Internationale Veröffentlichungsnummer: WO8901786

(56) Entgegenhaltungen:
- FR-A- 2 335 239
- US-A- 4 251 482
- US-A- 4 457 327

## Beschreibung

Die Erfindung bezieht sich auf ein verfahren zum Steuern eines Ventils für einen Sterilisierbehälter, und auf ein zur Durchführung des Verfahrens geeignetes Ventil. Derartige Ventile ermöglichen innerhalb eines Sterilisators den Medienaustausch in den Sterilisierbehälter hinein und aus diesem heraus. Der Ventilkörper dieses Ventils wird in der letzten Vakuumphase unter Aufrechterhaltung eines Unterdrucks im Behälter über einen Drucksensor und ein Temperaturglied gesteuert geschlossen.

Derartige Ventile sind beispielsweise aus der US-A-4 251 482 und US-A-4 228 914 bekannt. Sie haben gegenüber bekannten Doppelrückschlagventil-Anordnungen anderer Sterilisierbehälter den Vorteil, daß das Innere des Sterilisierbehälters bis zum Einlaufen in das Vakuum der Trocknungsphase mit dem Inneren der Sterilisatorkammer frei in Verbindung steht, so daß ständig ein Medienaustausch erfolgen kann. Vorteilhaft ist bei den druck- und temperaturabhängig gesteuerten Ventilen außerdem die Tatsache, daß nach Abschluß des Sterilisationsverfahrens im Inneren des Behälters ein Unterdruck bestehen bleibt.

Die US-A-4 251 482 / US-A-4 228 914 Zeigen Sterilisierbehälter mit einem im Boden angeordneten Ventil, welches über eine balgartige Expansionskammer geschlossen wird. Der schrumpfschlauch schließt eine nach dem Inneren des Balges führende Öffnung, und bei äußerem Druckabfall dehnt sich der Balg aus und schließt das Ventil. Das thermische Schrumpfen des Schlauches erfolgt während der Sterilisierphase zeitlich an einer stelle, die nicht genau definiert ist. Das Schließen des Ventils erfolgt,ebenfalls zeitlich nicht genau definiert, noch vor der Vakuumtrocknungstrocknungsphase. Der Dampf soll nicht durch das Ventil abgezogen werden, sondern durch den Behälterdeckel über die Dichtung, d.h. während der Vakuumtrockungsphase darf der Deckel nicht dicht abschließen, damit Dampf und Feuchtigkeit abgeführt werden können.

Bei dem in der US-A-4 251 482 beschriebenen Anordnung ist als Drucksensor ein Ausdehnungsgefäß vorgesehen, welches in der Dampfsterilisationsphase auf thermischem Wege durch einen Schrumpfschlauch geschlossen wird, der damit eine Eintrittsöffnung des Ausdehnungsgefäßes sperrt. Dies hat zur Folge, daß sich der Drucksensor beim Einlaufen in die Nachtrocknungs-Vakuumphase unter der Wirkung des ihm innewohnenden Überdrucks ausdehnt. Diese Ausdehnung wird zur Verschiebung eines Ventilkörpers in die Schließstellung benutzt, wodurch der Sterilisierbehälter abgedichtet wird. In der letzten Vakuumphase, d.h. während des Trockenvorganges bleibt das Ventil geschlossen und kann nicht mehr zum Medienaustausch beitragen.

Ein Absaugen der durch Rückverdampfung des Kondensats entstehenden Dampfphasen muß daher durch die Behälterdeckeldichtung erfolgen, oder es muß zusätzlich, wie in der US-A-4 228 914 beschrieben, ein weiteres Ventil vorgesehen werden, das öffnet wenn ein Vakuum außen an den Behälter angelegt wird, um so den Wasserdampf abziehen zu können.

Bei dem Sterilisierbehälter nach der FR-A-2 335 239 ist wiederum im Boden ein Ventil vorgesehen, welches in diesem Fall nicht durch einen Schrumpfschlauch, sondern über ein abschmelzendes Eutektikum in Schließstellung überführt wird. Es ist völlig unmöglich, daß hierbei das Schließen des Ventils in der Belüftungsphase erfolgt, weil das Eutektikum während der Sterilisierphase B schmilzt und nach dem Schmelzen das Ventil sogleich in den Schließzustand überführt wird. Die Trocknung kann auch hier wieder nur über die Deckeldichtung während der Vakuumtrocknungsphase erfolgen. Es trifft aber nicht zu, daß der Ventilkörper erst in der Belüftungsphase in die Schließstellung gesteuert wird. Insofern unterscheidet sich die Wirkungsweise des erfindungsgemäßen Ventils grundsätzlich von dem bekannten Ventil.

Der Erfindung liegt die Aufgabe zugrunde, ein Steuerverfahren für ein Ventil und ein Ventil für einen Sterilisierbehälter zu schaffen, das nicht nur in der Vorvakuumphase und in der Sterilisationsphase, sondern auch noch während der abschließenden Nachtrocknungs-Vakuumphase offen ist und erst nach Einleitung der Belüftungsphase schließt.

Gelöst wird die gestellte Aufgabe durch die im Kennzeichnungsteil des Verfahrensanspruchs 1 bzw. des Gegenstandsanspruchs 5 angegebenen Merkmale.

Das Wesen der vorliegenden Erfindung, worin sich diese grundsätzlich vom bekannten Stand der Technik unterscheidet, besteht demgemäß darin, daß (mit dem Ventil) der Behälter während der Belüftungsphase geschlossen wird. Hierdurch wird gewährleistet, daß die gesamte Vakuumphase für die Trocknung zur Verfügung steht, weil Feuchtigkeit noch bis in die Belüftungsphase entweichen kann.

Nach der Erfindung erfolgt das Schließen des Ventils durch Zusammenquetschen des das Ventil offenhaltenden Drucksensors nach Einleitung der Belüftungsphase durch den sich erhöhenden Außendruck, gegen den die den Drucksensor bildenden Druckkapsel durch das Temperaturglied gesteuert abgesperrt ist. Die thermische Steuerung erfolgt durch die Dampfsterilisations-Temperatur, jedoch ist es für die erfindungsgemäße Wirkung nicht entscheidend, wann genau während der Dampfsterilisationsphase das Temperaturglied das Schließen des Drucksensors bewirkt. Es ist allein erforderlich, daß es erst vor Erreichen des letzten Druckanstieges (Belüftungsphase) den Drucksensor schließt.

Dies kann beispielsweise dadurch geschehen, daß eine Legierung oder eine andere Masse bei der jeweils verwendeten Sterilisationstemperatur schmilzt, die das Drucksensorventil bis zum Erreichen dieser Temperatur in Offenstellung gehalten hat.

Der Drucksensor weist ein Rückschlagventil auf, welches eine Entlüftung des Drucksensors bei abfallendem Außendruck ermöglicht, bei Druckanstieg jedoch wieder sperrt, so daß ein einmal angelegtes Vakuum im Drucksensor gehalten wird.

Im Gegensatz zu bekannten Ventilanordnungen erfolgt keine Temperatursteuerung des Behälterventils, sondern die Temperatur steuerung bewirkt nur eine Vorbereitung des Drucksensors für den Ventilschließvorgang während der Belüftungsphase. Durch Einstellen einer Vorspannfeder, die das Behälterventil in Öffnungsstellung hält, kann die Höhe des Unterdrucks exakt festgelegt werden, bei dem das Ventil schließt.

Das erfindungsgemäße Ventil kann am Behälter oder am Behälterdeckel angeordnet werden, und zwar ist es zweckmäßigerweise lösbar mit diesem verbunden, damit es getrennt gereinigt und gegebenenfalls gegen ein anderes ausgetauscht werden kann, dessen Legierung einen anderen, einer anderen Sterilisiertemperatur angepaßten Schmelzpunkt besitzt.

Zusammengefaßt ergeben sich durch das erfindungsgemäße Ventil gegenüber vergleichbaren Ventilen die folgenden Vorteile:
Das den Medienaustausch zurlassende Behälterventil bleibt während des gesamten Sterilisationszyklus bis zum Druckanstieg bei der Belüftung geöffnet. Es wird also nicht, wie beim bekannten Stand der Technik, bereits beim Einlauf in die Trocknungsphase geschlossen, sondern bleibt offen, so daß ein optimales Absaugen des in der Vakuumphase zurückverdampfenden Kondensates aus dem Behälter heraus ohne Behinderung durch Strömungswiderstände, wie Filter oder anliegende Dichtungen, gewährleistet wird.

Das Schließen des Ventils erfolgt bei einem vorbestimmbaren Vakuum. Die Höhe des eingeschlossenen Vakuums ist über das Verhältnis wirksame Querschnitte und Federkräfte leicht einstellbar. Das im Behälter verbleibende Vakuum kann im Gegensatz zu bekannten Anordnungen demgemäß geringer eingestellt werden als das Vakuum der Trocknungsphase, welches etwa 100 mbar beträgt. Demgemäß ist die nach abgeschlossener Belüftung den Behälter belastende Druckdifferenz entsprechend geringer als beim Stand der Technik, bei dem praxisfremde entsprechend druckfeste Sonderkonstruktionen erforderlich sind.

Weiter wird durch das erfindungsgemäße Ventil mit Sicherheit das Eindringen von Luft oder Gasen, und damit auch von Keimen während des Transportes und der Lagerung verhindert. Das Schließen des Ventils erfolgt z.B. bei 800 mbar Kammerdruck, die ja zu diesem Zeitpunkt auch im Sterilisierbehälter herrschen, und dann erfolgt der restliche Druckanstieg auf 1000 mbar, der aber vom Behälter wegen des zusammengeschlossenen Ventils nicht mehr ausgeglichen werden kann, so daß die gesamte Ventilkappe durch den Differenzdruck von 200 mbar von außen angepreßt und dadurch dicht versiegelt wird.

Zum Begrenzen des Vakuums wäre theoretisch zwar auch die Verwendung einer gefederten Rückschlagklappe bzw. eines Rückschlagvnetils in den Behälter hinein mit festgelegtem Ansprechdruck von z.B. 200 mbar möglich. Bei einer Belüftung über ein derartiges Ventil auf 1000 mbar Außendruck wären dann auch 800 mbar Vakuum im Behälter eingestellt. Aber bei der Entnahme aus dem Sterilisator besteht dann die Gefahr der Unsterilität, wenn nicht durch entsprechende Konstruktion des Strömungsweges das Eindringen von Keimen in den Behälter sicher verhindert wird:
Am Ende der Belüftung befände sich das System dann im Gleichgewicht, d.h. die anliegende Druckdifferenz von 200 mbar genügte dann gerade nicht, um die Rückschlagklappe gegen die Federkraft zu öffnen. Steigt aber der Außendruck, z.B. barometrischer Schwankungen, während der Lagerung (bis zu 60 mbar) oder sinkt der Innendurck durch die Abkühlung des ca. 80°C heißen Sterilisierbehälters, dann würde sich die Rückschlagklappe kurz zum Druckausgleich öffnen und es bestünde die Gefahr einer Kontamination des sterilen Inhalts, obgleich ein möglicherweise noch verbleibendes, aber vermindertes Restvakuum dem Benutzer fälschlicherweise suggerieren würde, daß der Behälter dicht war. Diese Gefahr ist bei Anwendung des erfindungsgemäßen Ventils ausgeschaltet, weil das Ventil erst in der abschließenden Belüftungsphase bei einem einstellbaren Unterdruck geschlossen wird und dann durch den außen auf dem Behälter lastenden Atmosphärendruck bis zum Öffnen versiegelt bleibt. Die Anzeige des Vakuumanzeigers oder das Zischen beim Öffnen des Behälters durch die einströmende Luft sind ein sicheres Indiz dafür, daß der Behälter seit seiner Sterilisation bis zur Öffnung gasdicht und damit keimdicht war.

Zweckmäßige Ausgestaltungen der Erfindung ergeben sich aus den Unteransprüchen 2 bis 4 bzw. 6 bis 17.

Nachstehend werden Ausführungsbeispiele der Erfindung anhand der Zeichnung beschrieben. In der Zeichnung zeigen:

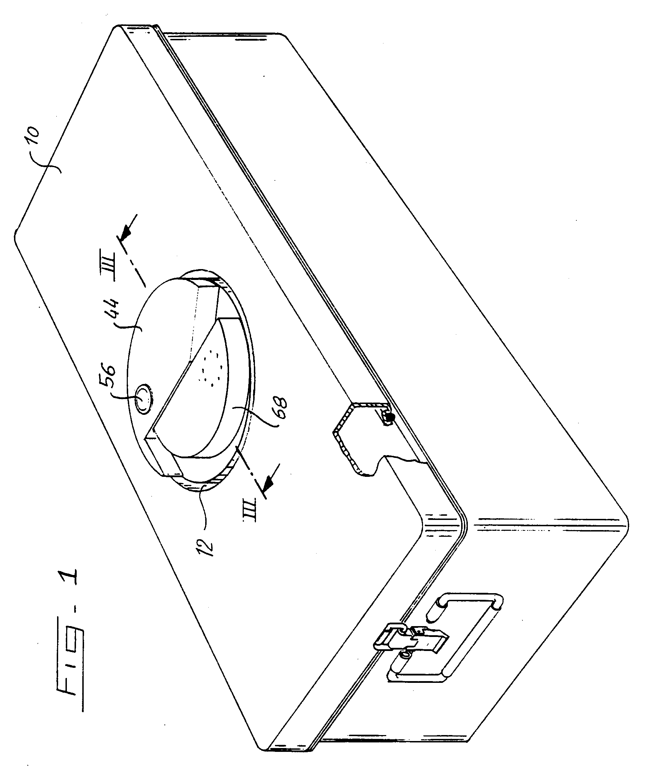

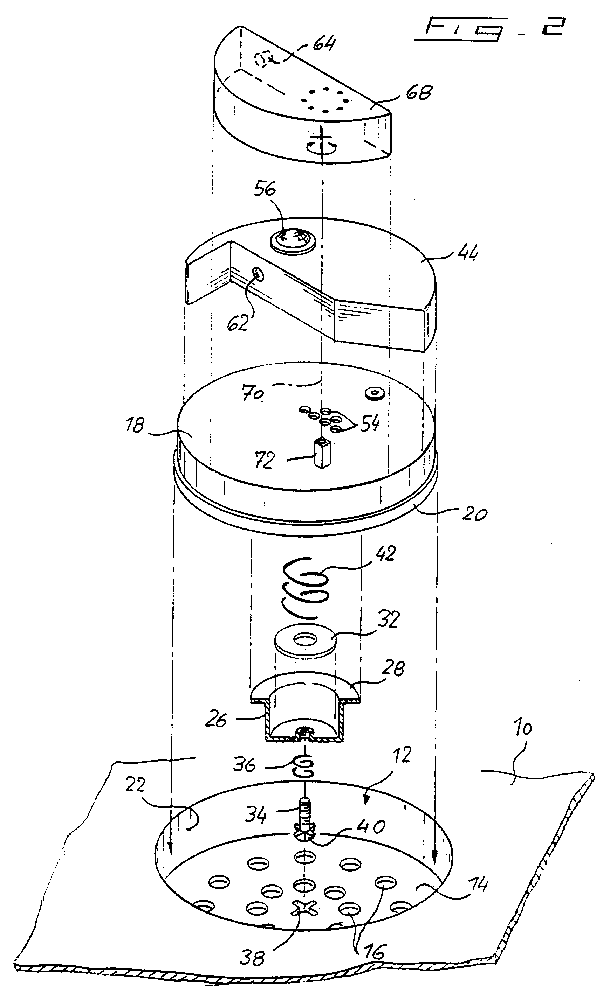

Fig. 1 eine perspektivische Ansicht eines Sterilisierbehälters mit einem erfindungsgemäß ausgebildeten Ventil im Deckel;
Fig. 2 eine auseinandergezogene perspektivische Darstellung des Ventils;
Fig. 3 einen Schnitt nach der Linie III-III gemäß Fig. 1 bzw. Fig. 4;
Fig. 4 einen Schnitt nach der Linie IV-IV gemäß Fig. 3;
Fig. 5 in größerem Maßstab einen Schnitt nach der Linie V-V gemäß Fig. 4;
Fig. 6 eine auseinandergezogene perspektivische Darstellung der Schaltvorrichtung für das Ventil;
Fig. 7 eine graphische Darstellung des zeitlichen Druckverlaufs und des zeitlichen Bewegungsablaufs.
Fig. 8 eine schematische Ansicht einer weiteren Ausführungsform eines im Deckel eines Sterilisierbehälters angeordneten thermisch gesteuerten Ventils.

Der Deckel (10) des Sterilisierbehälters weist im Mittelteil eine napfförmige Vertiefung (12) auf, deren Boden (14) mit Medienaustauschöffnungen (16) versehen ist. Innerhalb der napfförmigen Vertiefung (12) befindet sich eine Ventilkappe (18), die mit ihrer Ringdichtung (20) gegen den Boden (14) der Vertiefung in Schließstellung des Ventils abdichtet. Die Medienaustauschöffnungen (16) befinden sich radial innerhalb der von der Ringdichtung (20) umschlossenen Fläche des Bodens (14). Zwischen der Kappe bzw. der Dichtung (20) und der Seitenwand (22) der napfförmigen Vertiefung befindet sich ein zylindrischer Ringspalt (24), der genügend breit ist um den freien Medienaustausch bei geöffnetem Ventil zu gewährleisten.

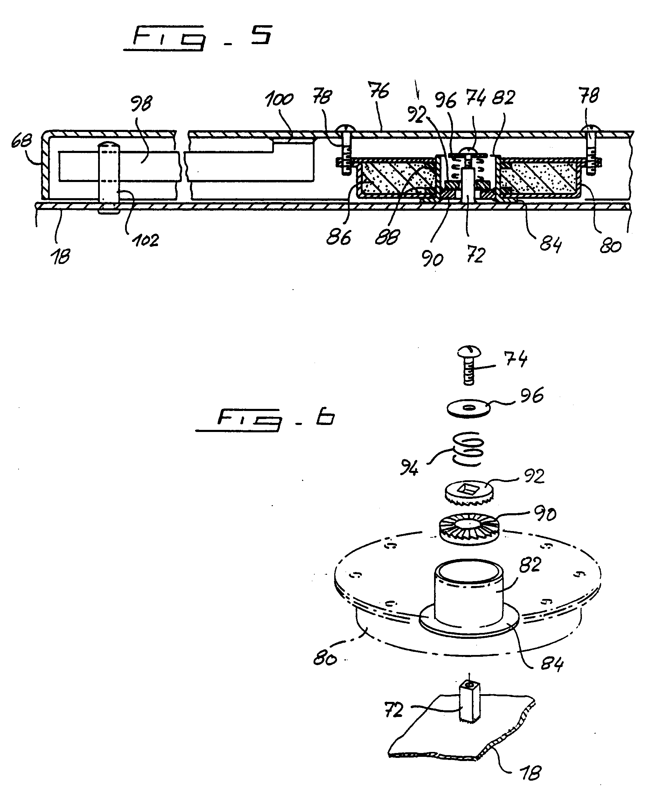

Die Deckelplate der Ventilkappe (18) trägt innen konzentrisch einen topfförmigen Stützkörper (26), der mit seinem Außenflansch (28) mittels eines Klemmrandes (30) der Ventilkappe dichtend gegen diese verspannt ist. Der Stützkörper (26) besteht aus elastischem Material und er ist in Abhängigkeit von Druckdifferenzen axial zusammendrückbar. Auf dem Boden des Stützkörpers ruht eine Ringscheibe (32), in deren Innengewinde eine Schraube (34) eingedreht ist, die von unten her durch ein Loch des Bodenteils des topfförmigen Stützkörpers (26) gesteckt ist. Die Schraube (34) ist mit dem Bodenteil, beispielsweise durch Verklebung dichtend verbunden. Auf die Schraube (34) ist eine Schraubenfeder (36) aufgezogen, die sich im zusammengebauten Zustand einerseits gegen den Boden des topfförmigen Stützkörpers (26) und andererseits gegen den Boden (14) der napfförmigen Vertiefung abstützt. Dieser Boden Weist einen zentralen Kreuzschlitz (38) auf, durch den von oben her der entsprechend kreuzartig geformte Schraubenkopf (40) der Schraube (34) her eingesteckt und durch Drehung fixiert ist. Im Inneren des topfförmigen Stützkörpers (26) befindet sich eine Druckschraubenfeder (42), die sich gegen die Ringscheibe (32) und den Deckel der Kappe (18) abstützt und den Stützkörper (26) axial ausspannt, so daß dieser Stützkörper bei fehlender Druckdifferenz die aus Fig. 3 ersichtliche Stellung einnimmt, in der er die Ventilkappe bzw. deren Ringdichtung (20) vom Boden (14) abhebt und dadurch das Ventil offenhält. Das Ventil schließt, wenn bei einem vorbestimmten Differenzdruck der Stützkörper (26) axial gegen die Kraft der Feder (42) zusammengequetscht wird.

Diese Funktion wird weiter unten im einzelnen noch beschrieben.

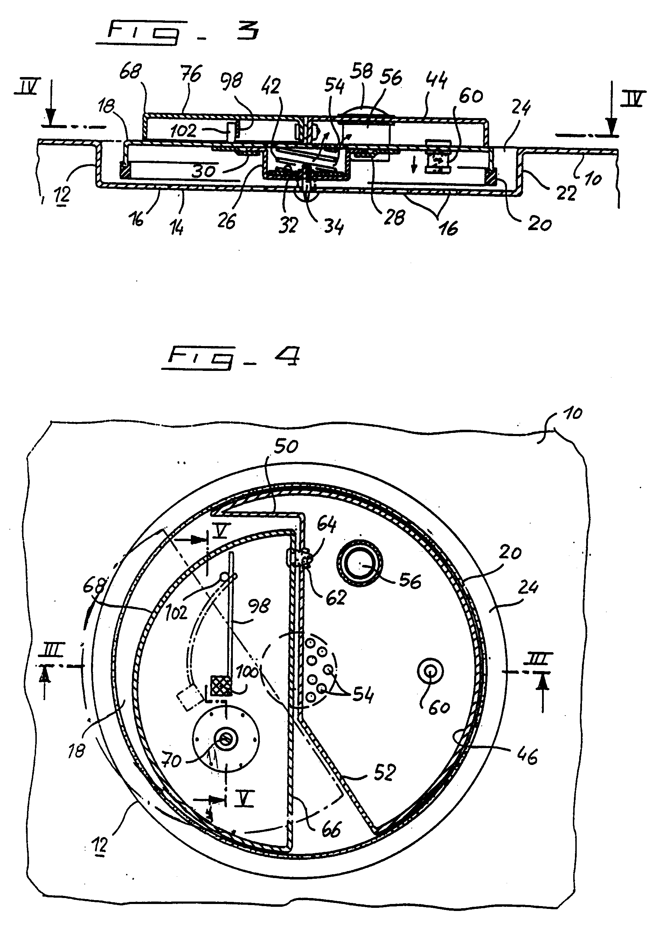

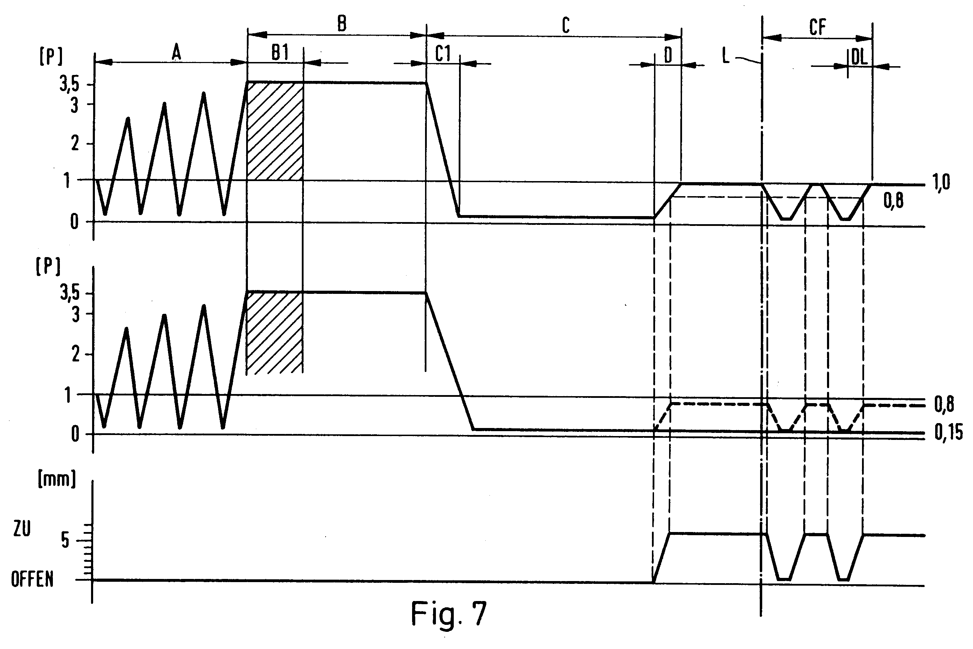

Die Ventilkappe (18) trägt einen hohlen Aufsatz (44), dessen Form am besten aus Fig. 2 ersichtlich ist. Gemäß dem dargestellten Ausführungsbeispiel weist die Seitenwand des Aufsatzes (44) einen über etwa 180° verlaufenden kreiszylindrischen Abschnitt (46) und einen, über einen Teil eines Durchmessers verlaufenden Abschnitt (48) sowie zwei Sehnenabschnitte (50 und 52) auf. Der erste Sehnenabschnitt (50) verläuft wie aus Fig. 4 ersichtlich, im rechten Winkel zu dem Durchmesserabschnitt (48) und verbindet diesen mit der Ringwand (46). Der zweite Sehnenabschnitt (52) springt, wie aus Fig. 4 ersichtlich, gegenüber dem Durchmesserabschnitt (48) zurück und bildet mit diesem einen stumpfen Winkel. Dieser Aufsatz (44) ist vakuumdicht auf der Ventilkappe (18) aufgesetzt oder einstückig mit dieser geformt. Durchgangslöcher (54) in der Ventilkappendeckelplatte verbinden das Innere des Aufsatzes (44) mit dem Inneren des topfförmigen Stützkörpers (26). Die Deckelplatte des Aufsatzes (44) trägt einen Vakuumanzeiger (56). Dieser weist eine dichtend eingespannte Membran (58) auf, die durch eine nicht dargestellte innere Feder nach außen aufgewölbt wird (wie in Fig. 2 und 3 dargestellt) und die nach innen eingezogen wird, sobald der Innenraum unter einem gegenüber dem Außendruck niedrigeren Druck steht.

Im Bereich des Aufsatzes (44) ist in der Deckelplatte der Ventilkappe (18) ein Rückschlagventil (60) eingesetzt, welches aus noch zu erläuternden Gründen eine Strömung aus dem Inneren des Aufsatzes (44) heraus, aber nicht umgekehrt, zuläßt.

Der Durchmesserwandabschnitt (48) weist eine Ventilöffnung (62) auf, die durch einen Stopfen (64) verschließbar ist. Dieser Stopfen (64) steht von der Durchmesserseitenwand (66) eines halbzylindrischen Stellgliedes (68) vor, das um eine bezüglich der Zylinderachse der Ventilkappe (18) exzentrische Achse (70) zwischen der in Fig. 4 voll ausgezogenen Schließstellung und der strichpunktiert eingezeichneten Öffnungsstellung verschwenkbar ist. Wie aus Fig. 4 ersichtlich, befindet sich das Stellglied (68) in seiner strichpunktierten Öffnungsstellung teilweise außerhalb der napfförmigen Vertiefung (12) und über der Ebene des Behälterdeckels, so daß in dieser Stellung ein Schließen der Ventilkappe verhindert ist. In dieser Öffnungsstellung wird in nachstehend beschriebener Weise das Stellglied gehalten, bis es temperaturgesteuert in die Schließstellung überführt wird. Die Arbeitsweise und die Schaltvorrichtung für das Stellglied (68) sind am besten aus den Figuren 5 und 6 ersichtlich:
Auf der Oberseite des Deckels der Ventilkappe (18) ist ein im Querschnitt polygonal gestalteter Zapfen (72) aufgeschweißt oder auf andere Weise fixiert. Der Zapfen besitzt eine in der Achse (70) liegende Gewindebohrung, in die eine Schraube (74) einschraubbar ist. Die Deckelplatte (76) des Stellgliedes (68) ist über Schrauben (78) mit eine Trommel (80) verbunden. Diese Trommel (80) umschließt eine Hülse (82), die konzentrisch zur Achse (70) liegt und mit ihrem Außenflansch (84) auf der Ventilkappe (18) abgestützt ist. Die nach der Hülse (82) hin offene Trommel (80) ist mit einer Masse (86) vorzugsweise einer eutektischen Metallegierung gefüllt, die bei einer vorbestimmten Sterilisationstemperatur, beispielsweise bei 121°C oder bei 134°C schmilzt.

Im erstarrten Zustand haftet das Eutektikum (86) an der Trommel (80) und an der Hülse (82) an bzw. ist es auf die Hülse aufgeschrumpft, so daß in diesem Zustand eine drehfeste Verbindung zwischen der Trommel (80) bzw. dem Stellglied (68) und der Hülse (82) vorhanden ist. Erforderlichenfalls kann die Hülse (86) nach außen weisende Vorsprünge in Gestalt von Stiften, Schrauben Buckeln oder dergleichen aufweisen, die eine formschlüssige Verbindung gewährleisten. Sobald das Eutektikum (86) auf die Sterilisiertemperatur von z.B. 121°C oder 134°C aufgeheizt ist, schmilztz es und die drehfeste Verbindung zwischen Trommel (80) und Hülse (82) wird aufgehoben. Ein Ausfließen des Eutektikums aus der Trommel (80) wird durch Dichtungsringe (88) verhindert.

Im Inneren der Hülse (82) ist eine Zahnscheibe (90) mit axialer Verzahnung fixiert, die den Zapfen (72) mit Spiel umschließt und gegenüber diesem frei drehbar ist. Auf dieser Zahnscheibe (90) liest eine weitere Zahnscheibe (92) so, daß ihre Verzahnungen axial ineinandergreifen. Die Verzahnungen der Zahnscheiben (90 und 92) sind als Sägeverzahnungen derart ausgebildet, daß eine Rutschkupplung entsteht, wie dies weiter unten beschrieben wird. Die Zahnscheibe (92) besitzt ein polygonales, dem Querschnitt des Zapfens (72) entsprechendes Loch und ist auf diesen Zapfen (72) drehfest aufgesteckt. Die Zahnscheiben (90 und 92) werden durch eine auf die Schraube (74) aufgezogene Druckschraubenfeder (94) gegeneinandergedrückt, die sich einerseits an der Zahnscheibe (92) und andererseits an einer am Kopf der Schraube (74) anliegenden Ringscheibe (96) abstützt.

Das Stellglied (68) ist durch eine Blattfeder (98) in Schließstellung vorgespannt. Diese Blattfeder (98) ist an einem Ende (100) an der Deckelplatte (76) des Stellgliedes (68) festgelegt und verläuft parallel zu dieser Deckelplatte nach außen. Das freie Ende der Blattfeder (98) liegt einem Stift (102) an, der von der Ventilkappe (18) nach oben vorsteht.

Bei erstarrtem Eutektikum (86) kann das Stellglied (68) von Hand aus der in Fig. 4 voll ausgezogenen Schließstellung in die in Fig. 4 strichpunktiert dargestellte Öffnungsstellung überführt werden, wobei die Feder (98) gespannt wird. In dieser Stellung ist, wie aus Fig. 4 ersichtlich, eine Schließbewegung des Ventils mechanisch blockiert, so daß das Ventil (18) nicht durch den dynamischen Druck des einströmenden Dampfes zugedrückt werden kann. Die mechanische Blockierung kommt dadurch zustande, daß das drehbare Stellglied über die Seitenwand (22) über den Deckelrand geschwenkt und hier festgehalten wird. Bei dem Spannvorgang des Stellgliedes wird über die hiermit fest verbundene Trommel (80) die Hülse (82) gemäß Fig. 4 im Gegenuhrzeigersinn verdreht, wobei die Verzahnungen der Zahnscheiben (90 und 92) aufeinander abrutschen können. In Gegenrichtung, d.h. im Uhrzeigersinn gemäß Fig. 4 erfolgt jedoch eine drehfeste Verbindung über die Verzahnungen,so daß das Stellglied in Öffnungsstellung gehalten wird weil die Zahnscheibe (92) auf dem an der Ventilkappe (18) fixierten Zapfen (72) drehfest sitzt. Gemäß dem dargestellten Ausführungsbeispiel steht das Ventil über die Oberfläche des Behälterdeckels vor. Um die Behälter stapelbar zu machen, müssen diese entweder Füße entsprechender Höhe besitzen oder das Ventil muß in einer weiteren Stufenvertiefung unter der Deckeloberfläche untergebracht werden.

Das erfindungsgemäße Ventil arbeitet wie folgt:
Der gesamte Ventilmechanismus, der in der napfförmigen Vertiefung (12) des Deckels des Sterilisierbehälters Aufnahme findet, ist leicht lösbar mit diesem zu verbinden. Es kann als getrennte Einheit gereinigt werden. Im sauberen Zustand wird es mit dem Sterilisierbehälter dadurch verbunden, daß der kreuzförmige Schraubenkopf (40) in den Kreuzschlitz (38) des Bodens (14) eingesteckt und die gesamte Ventileinheit bis zu einem Anschlag verdreht wird, wodurch der Schraubenkopf bajonettartig verriegelt wird. Nach dem Einsetzen bleibt das Ventil geöffnet, d.h. die Ringdichtung (20) behält einen Abstand vom Boden (14) bei. In diesem Zustand wird der Sterilisierbehälter in den Sterilisator gebracht und in der Anfangsphase A (vgl. Fig. 7) mehrfach evakuiert. Dann erfolgt eine Temperaturerhöhung bis zu einem Maximalbetrag, der je nach Art des zu sterilisierenden Gutes, z.B. bei 121°C oder bei 134°C liegt. Die Einstellung auf die jeweilige Sterilisiertemperatur kann durch Austausch der Ventilanordnung erfolgen. Für unterschiedliche Sterilisiertemperaturen wird eine jeweils bei dieser Temperatur schmelzende Legierung (86) verwendet.

Während der Vorvakuumphase A bleibt das Eutektikum (86) fest und das Stellglied (68) in der aus Fig. 4 strichpunktiert angedeuteten Lage. Damit bleibt das Hilfsventil (62, 64) offen und es kann ständig ein Druckausgleich Sensor-Sterilisierkammer erfolgen, so daß sich der zum Schließen der Ventilkappe (18) erforderliche Druck nicht aufbauen kann.

In einem ersten Abschnitt B 1 der Sterilisierphase B
schmilzt das Eutektikum (86) und dadurch wird die Reibungsverbindung bzw. die Formschlußverbindung zwischen dem Eutektikum (86) und der Hülse (82) aufgehoben. Dadurch wird das vorgespannte Stellglied aus der in Fig. 4 strichpunktiert dargestellten Öffnungsstellung durch die Feder (98) in die in Fig. 4 ausgezogen dargestellte Schließstellung überführt, in der der Stopfen (64) die Ventilöffnung (62) schließt. Damit ist der Innenraum des Aufsatzes (44) mit dem Innenraum des topfförmigen Stützkörpers gegenüber der äußeren Atmosphäre abgeschlossen. Über das Rückschlagventil (60) kann die Strömung jetzt nur noch aus der vom Aufsatz (44) gebildeten Kammer nach außen strömen.

Nach Abschluß der Sterilisierphase B sinkt in einem ersten Abschnitt Cl der Trocknungsphase C der Außendruck ab und es erfolgt ein über das Rückschlagventil (60) die Evakuierung des Drucksensors auf nahezu Kammerdruck. Die Ventilkappe (18) befindet sich jedoch immer noch in Öffnungsstellung. In die Schließstellung wird die Ventilkappe in der Belüftungsphase D dadurch überführt, daß der Außendruck wieder ansteigt, ein Druckausgleich über das Rückschlagventil jedoch nicht erfolgen kann, so daß innerhalb der Kammer und innerhalb des Stützkörpers (26) ein geringerer Druck verbleibt und der Stützkörper zusammenbricht und die Ventilkappe (18)sich dichtend anlegt. Im Gegensatz zu anderen Ventilanordnungen arbeitet das erfindungsgemäße Ventil auch dann ordnungsgemäß, wenn in einer fraktionierten Nachtrocknungsphase CF wiederholt feuchte Luft aus dem Behälter abgesaugt und durch trockene ersetzt wird, d.h. wenn in der Trockungsphase Druckänderungen auftreten. Dann erfolgt nämlich ein wiederholtes Öffnen und Schließen, und das Ventil wird in diesem Fall durch die letzte Belüftungsphase DL bei einer vorbestimmten Druckdifferenz geschlossen. Diese fraktionierte Nachtrocknungsphase ist in Fig. 7 rechts der strichpunktierten Linie L angedeutet.

In Fig. 7 stellt das obere Diagramm den Druck in der Sterilisatorkammer dar, das mittlere Diagramm den Druck im Sensor (ausgezogen) dargestellt und der Druck im Behälter (ab D strichliert dargestellt) und das untere Diagramm kennzeichnet die Ventilstellung.

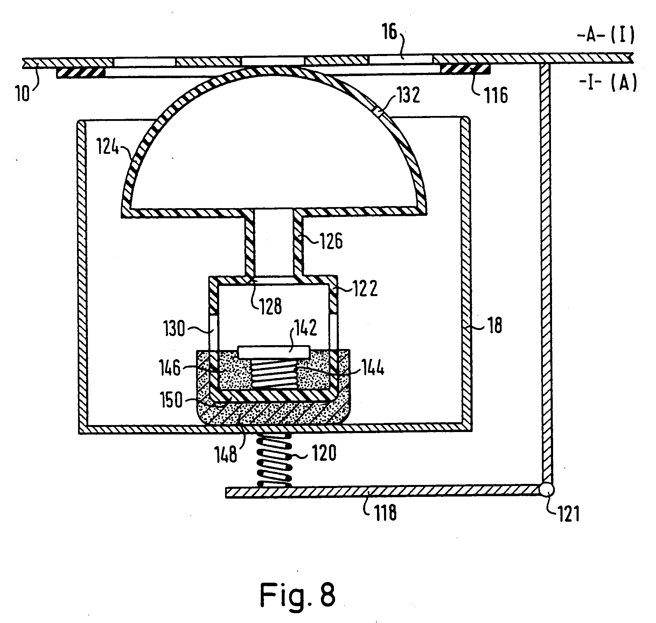

In Fig. 8 ist ein Abschnitt des Behälterdeckels (10) eines in bekannter Weise ausgebildeten Sterilisierbehälters dargestellt. Der Deckel (10) ist mit Medienaustauschöffnungen (16) versehen. Mit dem Bezugszeichen A ist die Außenseite des Behälters bezeichnet und mit dem Bezugszeichen I die Innenseite des Behälters, d.h. das Ventil ist an der Innenseite des Deckels angeordnet. Es könnte jedoch auch außerhalb des Deckels vorgesehen werden.

Rings um die Medienaustauschöffnungen (16) verläuft im Deckelinneren als Ventilsitz ein Dichtring (116), auf den eine Ventilkappe (18) durch eine Feder (120) aufpreßbar ist, die gegen
einen Schwenkhebel (118) abgestützt ist. Der Schwenkhebel (118) ist in der in der Zeichnung dargestellten Lage arretierbar und kann um seine Gelenkachse (121) nach innen verschwenkt werden.

Zum Offenhalten des Ventils in der dargestellten Öffnungsstellung dient eine Druckkapsel (124) aus Kunststoff, die bei Anliegen einer vor bestimmten Druckdifferenz in sich zusammenbricht (implodiert) und damit den Weg zum Schließen des Ventils unter der Wirkung der Feder (120) freimacht. Die nur einmal verwendbare Druckkapsel (124) ist pilzartig ausgebildet und weist eine Drosselöffnung (132) auf, durch die ein verzögerter Druckausgleich erfolgen kann. Der Stiel der pilzförmigen Druckkapsel (124) in Gestalt eines Rohrfortsatzes (126) steht mit einem Käfig (122) in Verbindung, der seitliche Durchlaßöffnungen (130) und einen Ventilsitz (128) am unteren Ende des Rohrfortsatzes (126) aufweist. Mit dem Ventilsitz (128) wirkt ein Ventilkörper (142) zusammen, der unter der Wirkung einer Ventilfeder (144) steht, die den Ventilkörper (142) in die Schließstellung vorspannt. Die Schließbewegung des Ventilkörpers (142) wird jedoch durch eine der jeweiligen Sterilisiertemperatur angepaßte thermische Plombe (146)verhindert, die in dem unteren napfförmigen Teil (150) des Käfigs (122) eingegossen ist und den Ventilkörper in der Offenstellung hält. Diese Plombe besteht aus einem Material, das bei einer vorbestimmten Temperatur schmilzt und dann den Ventilkörper freigibt, so daß er die Kapsel (124) schließen kann. Um eine zeitliche Verzögerung der Schließbewegung zu bewirken und um das Schließen des Ventils und damit das Zusammenbrechen der Druckkapsel (124) in den Bereich der Belüftungsphase zu verlegen, ist der untere napfförmige Teil (150) von einem ebenfalls napfförmigen Isolierkörper (148) umgeben, der die Temperatur zeitverzögert zu der thermischen Plombe gelangen läßt, die aus einer Kunststoffmasse oder einer Legierung bestehen kann.

Das erfindungsgemäße Ventil arbeitet wie folgt:
In der Vorvakuumphase kann ein freier Medienaustausch über die Durchlaßöffnungen (130), den abgehobenen Ventilkörper (18) und die Medienaustauschöffnungen (16) stattfinden. Dadurch bleibt die Druckkapsel (124) in ihrer in der Zeichnung dargestellten Form erhalten und stützt sich oberseitig am Deckel (10) und unten mit ihrem Isolierkörper (148) an der Ventilkappe (18) ab. Hierdurch wird das Ventil gegen die Wirkung der Schließfeder (120) in Offenstellung gehalten. Diese Offenstellung bleibt auch während der Sterilisierphase aufrecht erhalten, während der die Sterilisierkammer einem erhöhten Druck und einer erhöhten Temperatur ausgesetzt wird. Diese erhöhte Temperatur bewirkt zeitverzögert über den thermischen Isolierkörper (148) eine allmähliche Erwärmung der thermischen Plombe (146). Der Wärmedurchgang durch den Isolierkörper und den napfförmigen Teil (150) sowie der Erweichungs- bzw. Schmelzpunkt der thermischen Plombe (146) sind derart aufeinander abgestimmt, daß das Hilfsventil (142) in der Vorvakuumphasew (A) nicht geschlossen werden kann, sondern erst am Anfang (B1) der Sterilisierphase (B). Druckunterschiede in der Vakuumphase können zeitverzögert durch die Drosselöffnung (132) ausgeglichen werden. In der letzten Vakuumphase ist daher auch bei geschlossenem Ventil (142) der Innendruck der Kapsel (124) gleich dem Umgebungsdruck. In der Belüftungsphase erfolgt der Druckanstieg so schnell, daß sich trotz der Drosselöffnung (132) eine erhebliche Druckdifferenz über der Druckkapsel (124) aufbauen kann, so daß diese Kapsel infolge einer nun auf ihr lastenden vorbestimmten Druckdifferenz zusammenbricht, d.h. implodiert und dadurch die Ventilkappe (18) freigibt, die dann auf den Ventilsitz (116) durch die Feder (120) gedrückt wird,so daß der Behälter nunmehr unter Aufrechterhaltung eines vorbestimmten Vakuums im Sterilisierbehälter abgedichtet ist.

## Patentansprüche

1. Verfahren zum Steuern eines Ventils für einen Sterilisierbehälter, das innerhalb eines Sterilisators den Medienaustausch in den Behälter hinein und aus diesem heraus ermöglicht und wenigstens bis zum Abschluß der Sterilisierphase offen ist, und dessen Ventilkörper (18) in der an die Sterilisierphase (B) anschließenden Trocknungsphase (C) über einen Drucksensor (26, 44) und ein Temperaturglied (86) gesteuert schließt, dadurch gekennzeichnet, daß der Ventilkörper (18) durch den Drucksensor (26, 42, 44; 124) gegen eine vorbestimmbare Kraft in Offenstellung gehalten wird, der über das Temperaturglied (64, 68, 86; 146) gesteuert den Ventilkörper (18) und damit den Sterilisierbehälter in der Belüftungsphase (D) bei einem vorbestimmbaren Unterdruck schließt.

2. Verfahren nach Anspruch 1,
dadurch gekennzeichnet, daß beim Erreichen des Sterilisationsdruckes während der Sterilisierphase (B) eine Druckkapsel derart abgedichtet wird, daß nur ein Druckausgleich von innen nach außen erfolgen kann und daß in der Belüftungsphase (D) im Sterilisator die Kapsel durch den sich aufbauenden Differenzdruck kontinuierlich zusammengedrückt wird, bis bei Erreichen des vorbestimmten Druckes das Ventil und damit der Sterilisierbehälter geschlossen werden und der weitere Druckanstieg auf Atmosphärendruck dann als Schließdruck auf dem Ventilkörper (18) lastet.

3. Verfahren nach Anspruch 1 und 2,
dadurch gekennzeichnet, daß das Abdichten der Druckkapsel (44) über ein Hilfsventil (62, 64) in Abhängigkeit von der Sterilisiertemperatur erfolgt.

4. Verfahren nach den Ansprüchen 1 bis 3,
dadurch gekennzeichnet, daß durch die manuelle Öffnung des Hilfsventils (62, 64) ein Druckausgleich Atmosphäre-Drucksensorinnenraum und über ein gegebenenfalls mit einem auswechselbaren Filter versehenen Rückschlagventil (60) ein Druckausgleich Drucksensorinnenraum-Behälterinnenraum erfolgt und gleichzeitig über eine in einer Richtung wirkende Kupplung ein Stellglied (76) in die Ausgangsstellung vorgespannt (98) wird.

5. Ventil zur Durchführung des Verfahrens gemäß Anspruch 1-4,
dadurch gekennzeichnet, daß der als Ventilkappe (18) ausgebildete Ventilkörper durch einen aus elastischem Material bestehenden, topfförmigen, durch eine Feder (42) aufgespannten Drucksensor (26) in Öffnungsstellung abgestützt ist, und daß dieser Drucksensor (26) mit dem Inneren eines Ventilaufsatzes (44) in Verbindung steht, der ein Hilfsventil mit einer Ventilöffnung (62) aufweist, die durch einen Stopfen (64) eines Temperaturstellgliedes (68, 86) absperrbar ist.

6. Ventil nach Anspruch 5,
dadurch gekennzeichnet, daß der Ventilaufsatz aus einer in sich starren Druckdose (44) besteht, die mit wenigstens einem axial zusammendrückbaren Stützkörper (26) in Verbindung steht, die den Ventilkörper (18) in Offenstellung halten.

7. Ventil nach den Ansprüchen 5 und 6,
dadurch gekennzeichnet, daß die Druckdose (44) über ein in der Deckelplatte der Ventilkappe (18) eingesetztes Rückschlagventil (60) mit der Druckkammer des Sterilisators verbunden ist.

8. Ventil Fach den Ansprüchen 5 bis 7,
dadurch gekennzeichnet, daß das Temperaturstellglied (68) auf der Ventilkappe (18) drehbar gelagert und in die Ventilschließstellung vorgespannt (98) ist, daß eine immer in einer Drehrichtung wirkende Kupplung (90, 92) das Temperatur-Stellglied (68) in Schließrichtung sperrt, jedoch eine manuelle Öffnung zuläßt, und daß die Kupplung durch ein Thermoglied (86) des Temperatursensors überbrückt ist, damit das Stellglied bei geschmolzenem Thermoglied in die Schließstellung ablaufen kann.

9. Ventil nach Anspruch 8,
dadurch gekennzeichnet, daß das Stellglied (68) eine Trommel (80) koaxial zu seiner Drehachse (70) aufweist, in die eine Hülse (82) als Abtriebsglied der Kupplung einsteht, und daß eine bei einer vorbestimmten Temperatur schmelzende Legierung (86) im festen Zustand, also vor und nach der Sterilisierphase (B), Trommel (80) und Hülse (82) drehfest verbindet.

10. Ventil nach den Ansprüchen 5 bis 9,
dadurch gekennzeichnet, daß die Ventilkappe (18) mit Drucksensor (26, 44) und Temperaturglied (68, 86) lösbar (34, 38, 40) am Behälter oder Behälterdeckel festlegbar ist.

11. Ventil nach Anspruch 10,
dadurch gekennzeichnet, daß die Ventilkappe (18) in einer napfförmigen Vertiefung (12) des Behälterdeckels (10) angeordnet ist, in deren Boden (14) die Medienaustauschöffnungen (16) angeordnet sind.

12. Ventil nach Anspruch 8,
dadurch gekennzeichnet, daß das Stellglied (68) in Offenstellung das Schließen der Ventilkappe (18) blockiert.

13. Ventil zur Durchführung des Verfahrens gemäß einem der Ansprüche 1 bis 3,
dadurch gekennzeichnet, daß der Ventilkörper als Kappe (18) ausgebildet ist und daß der Drucksensor als Druckkapsel (124) ausgebildet ist, die durch ein Temperaturglied (142, 146) absperrbar ist und bei der Belüftung des Sterilisators bei Erreichen eines vorbestimmten Differenzdruckes zusammenbricht und die Ventilkappe zum Schließen freigibt.

14. Ventil nach Anspruch 13,
dadurch gekennzeichnet, daß das Temperaturglied eine Thermoplombe (146) ist.

15. Ventil nach Anspruch 13 und 14,
dadurch gekennzeichnet, daß die Druckkapsel (124) einen Druckausgleichskanal (126) aufweist, der durch das thermisch gesteuerte Ventil (142, 146) absperrbar ist.

16. Ventil nach einem der vorhergehenden Ansprüche 13-15,
dadurch gekennzeichnet, daß ein Zeitverzögerungsglied in Form eines Wärmeisolators (148) Vorgesehen ist, das die Thermoplombe (146) zeitverzögert erwärmt.

17. Ventil nach den Ansprüchen 13-16,
dadurch gekennzeichnet, daß die Druckkapsel (124) als austauschbarer Einwegkörper ausgebildt ist.

## Claims

1. Method for controlling a valve for a sterilisation container, which permits, inside a steriliser, the exchange of media into the container and from the latter, and is open at least until the sterilisation phase is complete, and the valve body (18) of which, in the drying phase (C) following the sterilisation phase (B) closes under the control of a pressure sensor (26, 44) and a temperature element (86), characterised in that the valve body (18) is maintained in the open position, counter to a pre-determinable force, by the pressure sensor (26, 42, 44; 124) which, under the control of the temperature element (64, 68, 86; 146), closes the valve body (18), and hence the sterilisation container, in the ventilation phase (D) at a pre-determinable reduced pressure.

2. Method according to Claim 1, characterised in that, when the sterilisation pressure is reached during the sterilisation phase (B), a pressure capsule is sealed off in such a way that a pressure compensation can take place only from inside to outside, and in that, in the ventilation phase (D) in the steriliser, the capsule is continuously compressed by the build-up in differential pressure, until, when the pre-determined pressure is reached, the valve and hence the sterilisation container are closed and the further increase in pressure to atmospheric pressure is then exerted as a closing pressure on the valve body (18).

3. Method according to Claim 1 and 2, characterised in that the pressure capsule (44) is sealed off by an auxiliary valve (62, 64) as a function of the sterilisation temperature.

4. Method according to Claims 1 to 3, characterised in that, as a result of manually opening the auxiliary valve (62, 64) a pressure compensation takes place between the atmosphere and the interior of the pressure sensor and, via a non-return valve (60) which is optionally provided with an exchangeable filter, a pressure compensation takes place between the interior of the pressure sensor and the interior of the container, and simultaneously a regulating element (76) is pre-tensioned (98) into the starting position via a unidirectionally acting coupling.

5. Valve for carrying out the method according to Claims 1-4, characterised in that the valve body designed as a valve cap (18) is supported in the open position by a pressure sensor (26) which consists of resilient material, has a pot shape and is tensioned by a spring (42), and in that this pressure sensor (26) communicates with the inside of a valve top (44) which has an auxiliary valve with a valve opening (62) which can be blocked off by a plug (64) of a temperature-regulating element (68, 86).

6. Valve according to Claim 5, characterised in that the valve top consists of an inherently rigid pressure cell (44) which communicates with at least one axially compressible supporting body (26) which maintains the valve body (18) in the open position.

7. Valve according to Claims 5 and 6, characterised in that the pressure cell (44) is connected to the pressure chamber of the steriliser via a non-return valve (60) set in the cover plate of the valve cap (18).

8. Valve according to Claims 5 to 7, characterised in that the temperature-regulating element (68) is mounted rotatably on the valve cap (18) and is pre-tensioned (98) into the valve-closing position, in that a coupling (90, 92), always acting in one direction of rotation, blocks the temperature-regulating element (68) in the closing direction, but permits manual opening, and in that the coupling is spanned by a thermoelement (86) of the temperature sensor, so that, when the thermoelement has melted, the regulating element can move into the closing position.

9. Valve according to Claim 8, characterised in that the regulating element (68) has a drum (80) coaxial with its axis of rotation (70), into which drum a sleeve (82) projects as the driven element of the coupling, and in that an alloy (86) which melts at a pre-determined temperature, in the solid state, in other words before and after the sterilisation phase (B), rigidly connects the drum (80) and sleeve (82).

10. Valve according to Claims 5 to 9, characterised in that the valve cap (18), with the pressure sensor (26, 44) and temperature element (68, 86), can be fixed detachably (34, 38, 40) on the container or on the cover of the container.

11. Valve according to Claim 10, characterised in that the valve cap (18) is arranged in a bowl-shaped depression (12) of the cover (10) of the container, the media-exchange openings (16) being arranged in the base (14) of the depression.

12. Valve according to Claim 8, characterised in that the regulating element (68) blocks the closure of the valve cap (18) in the open position.

13. Valve for carrying out the method according to one of Claims 1 to 3, characterised in that the valve body is designed as a cap (18), and in that the pressure sensor is designed as a pressure capsule (124) which can be blocked off by a temperature element (142, 146) and collapses during the ventilation of the steriliser when a pre-determined differential pressure is reached, and allows the valve cap to close.

14. Valve according to Claim 13, characterised in that the temperature element is a thermal seal (146).

15. Valve according to Claim 13 and 14, characterised in that the pressure capsule (124) has a pressure-compensation passageway (126) which can be blocked off by the thermally controlled valve (142, 146).

16. Valve according to one of the preceding claims 13-15, characterised in that a time-delay element in the form of a thermal insulator (148) is provided, which heats up the thermal seal (146) in a time-delayed manner.

17. Valve according to Claims 13-16, characterised in that the pressure capsule (124) is designed as an exchangeable disposable body.

## Revendications

1. Procédé pour commander une soupape qui est destinée à un récipient de stérilisation, qui, à l'intérieur d'un stérilisateur, permet l'échange entre des milieux entrant dans le récipient et sortant de celui-ci, qui est ouverte au moins jusqu'à la fin de la phase de stérilisation, et dont le corps de soupape (18), dans la phase de séchage (C) qui suit immédiatement la phase de stérilisation (B), se ferme en étant commandée par l'intermédiaire d'un capteur de pression (26, 44) et d'un organe thermométrique (86), caractérisé par le fait que le corps de soupape (18) est maintenu en position d'ouverture par le capteur de pression (26, 42, 44 ; 124) à l'encontre d'une force pouvant être prédéterminée qui, par l'intermédiaire de l'organe thermométrique (64, 68, 86 ; 146), ferme de manière commandée le corps de soupape (18), et donc le récipient de stérilisation, dans la phase d'aération (D) pour une dépression pouvant être prédéterminée.

2. Procédé selon la revendication 1, caractérisé par le fait que, lorsque la pression de stérilisation est atteinte pendant la phase de stérilisation (B), une capsule manométrique est rendue étanche de telle manière qu'un équilibrage de la pression ne peut avoir lieu que de l'intérieur vers l'extérieur, et par le fait que, dans la phase d'aération (D) dans le stérilisateur, la capsule est comprimée continûment par la pression différentielle qui s'établit, jusqu'à ce que la soupape, et donc le récipient de stérilisation, soient fermés lorsqu'une pression prédéterminée est atteinte, et que la suite de l'augmentation de la pression jusqu'à la pression atmosphérique agit alors comme pression de fermeture sur le corps de soupape (18).

3. Procédé selon la revendication 1 et 2, caractérisé par le fait que la capsule manométrique (44) est rendue étanche par l'intermédiaire d'une soupape auxiliaire (62, 64) en fonction de la température de stérilisation.

4. Procédé selon les revendications 1 à 3, caractérisé par le fait qu'un équilibrage de pression entre l'atmosphère et le volume intérieur du capteur de pression a lieu grâce à l'ouverture manuelle de la soupape auxiliaire (62, 64), et qu'un équilibrage de pression entre le volume intérieur du capteur de pression et le volume intérieur du récipient a lieu par l'intermédiaire d'un clapet antiretour (60), lequel est muni le cas échéant d'un filtre interchangeable, et qu'en même temps un organe de commande (76) est rappelé (98) vers la position de départ par l'intermédiaire d'un accouplement agissant dans une direction.

5. Soupape pour la mise en oeuvre du procédé selon la revendication 1 à 4, caractérisée par le fait que le corps de soupape réalisé sous la forme d'un capuchon de soupape (18) est soutenu en position d'ouverture par un capteur de pression (26) en forme de pot qui est constitué par une matière élastique et qui est rappelé par un ressort (42), et par le fait que ce capteur de pression (26) est en communication avec l'intérieur d'un chapeau de soupape (44) qui comporte une soupape auxiliaire munie d'une ouverture de soupape (62), laquelle peut être fermée par un bouchon (64) d'un organe de commande thermométrique (68, 86).

6. Soupape selon la revendication 5, caractérisée par le fait que le chapeau de soupape se compose d'une boîte manométrique (44) qui est rigide en elle-même et qui est en communication avec au moins un corps d'appui (26), celui-ci pouvant être comprimé axialement et maintenant le corps de soupape (18) en position d'ouverture.

7. Soupape selon les revendications 5 et 6, caractérisée par le fait que la boîte manométrique (44) est reliée à la chambre de pression du stérilisateur par l'intermédiaire d'un clapet antiretour (60) inséré dans la plaque qui sert de couvercle au capuchon de soupape (18).

8. Soupape selon les revendications 5 à 7, caractérisée par le fait que l'organe de commande thermométrique (68) est monté tournant sur le capuchon de soupape (18), et qu'il est rappelé (98) vers la position fermée de la soupape, par le fait qu'un accouplement (90, 92) agissant toujours dans la direction de rotation bloque l'organe de commande thermométrique (68) dans la direction de la fermeture en autorisant toutefois une ouverture manuelle, et par le fait que l'accouplement est en parallèle avec un organe thermométrique (86) du capteur de température, afin que l'organe de commande puisse passer dans la position de fermeture lorsque l'organe thermométrique a fondu.

9. Soupape selon la revendication 8, caractérisée par le fait que l'organe de commande (68) présente un tambour (80) qui est coaxial avec son axe de rotation (70) et dans lequel pénètre un manchon (82) servant d'organe d'entraînement de l'accouplement, et par le fait qu'un alliage (86) qui fond à une température prédéterminée rend le tambour (80) et le manchon (82) solidaires en rotation lorsqu'il est à l'état solide, donc avant et après la phase de stérilisation (B).

10. Soupape selon les revendications 5 à 9, caractérisée par le fait que le capuchon de soupape (18) peut être monté de manière amovible (34, 38, 40) sur le récipient ou sur le couvercle du récipient avec le capteur de pression (26, 44) et l'organe thermométrique (68, 86).

11. Soupape selon la revendication 10, caractérisée par le fait que le capuchon de soupape (18) est disposé dans un renfoncement (12) en forme de cuvette du couvercle (10) du récipient, dans le fond (14) duquel sont disposés les orifices (16) d'échange entre les milieux.

12. Soupape selon la revendication 8, caractérisée par le fait que l'organe de commande (68) bloque en position d'ouverture la fermeture du capuchon de soupape (18).

13. Soupape pour la mise en oeuvre du procédé selon l'une des revendications 1 à 3, caractérisée par le fait que le corps de soupape est réalisé sous la forme d'un capuchon (18), et par le fait que le capteur de pression est réalisé sous la forme d'une capsule manométrique (124) qui peut être fermée par un organe thermométrique (142, 146) et qui, lors de l'aération du stérilisateur, s'affaisse et libère le capuchon de soupape en vue de la fermeture lorsqu'une pression différentielle prédéterminée est atteinte.

14. Soupape selon la revendication 13, caractérisée par le fait que l'organe thermométrique est un fusible thermique (146).

15. Soupape selon la revendication 13 et 14, caractérisée par le fait que la capsule manométrique (124) comporte un canal d'équilibrage de la pression (126) qui peut être fermé par la soupape à commande thermique (142, 146).

16. Soupape selon l'une des revendications précédentes 13 à 15, caractérisée par le fait qu'il est prévu un organe retardateur sous la forme d'un isolateur thermique (148) qui chauffe le fusible thermique (146) avec un retard dans le temps.

17. Soupape selon les revendications 13 à 16, caractérisée par le fait que la capsule manométrique (124) est réalisée sous la forme d'un corps jetable et interchangeable.
